# EUROPEAN PATENT APPLICATION

(11) **EP 1 754 464 A1**
(43) Date of publication of application: **21.02.2007**
(21) Application number: 05017845.8
(22) Date of filing: 17.08.2005
(51) Int. Cl.: A61K 6/033, A61K 38/43

(54) **Enzyme comprising dental composition**

(71) Applicant: 3M Innovative Properties Company, St. Paul, Minnesota 55144-1000 (US)
(72) Inventor: Kappler, Oliver Dr., 82362 Weilheim (DE); Häberlein, Ingo Dr., 82362 Weilheim (DE)
(74) Representative: Wallner, Astrid

(57) **Abstract**

The present invention relates to a dental composition comprising an enzyme having an enzyme activity at acidic pH values, a buffer providing an acidic pH value and one or more preservative agents, wherein at least one preservative agent is active at acidic pH.

## Description

The present invention relates to an enzyme comprising dental composition comprising of an enzyme active at acidic pH, a buffer providing an acidic pH and a preservative agent. Furthermore, the present invention relates to a process of producing this enzyme comprising dental composition. The invention also relates to the use of enzyme comprising dental compositions for producing a treatment agent for removing caries.

Caries which is also called tooth decay is one of the most frequently occuring human diseases. Caries is a bacterial damage of the tooth which may even cause teeth to fall out. From the outside, teeth are protected by a cover of hard enamel enclosing the softer dentin which in turn encloses the so-called pulp. The enamel itself consists of about 95 % inorganic compounds, especially hydroxyapatite, and about 5 % organic compounds and water. Dentin is softer than enamel and consists of about 65 % inorganic compounds (mainly hydroxyapatite), about 20 % organic compounds (mainly collagen and polysaccharides) and about 15 % water.

Caries develops in several steps by bacterial fermentation of carbohydrates, in particular by bacterial fermentation of sugar to acids. The acids resulting from said bacterial fermentation firstly dissolve the hard enamel, whereas bacteria attack mainly the organic components such as food particles having remained on the teeth.

If the enamel becomes porous and soft by the bacteria induced influence of acids, bacteria may reach the dentin layer below the enamel and infect it with caries. The region where the enamel or enamel and dentin are dissolved the most is called the caries lesion or cavity. The development of shallow cavities often continues and causes infection of the deeper parts of the tooth. These so called caries profunda situations show an extended area of caries infected dentin with the risk that the caries disease develops an inflammation of the pulp under the dentin. An inflammation of the pulp is extremely painful and may cause a serious risk to the health of the patient if it is not treated quickly.

The removal of the inflamed pulp in following endodontic treatments includes a disinfection of the root canal where the pulp is removed from. If this disinfection is not executed properly the infection may persist and may even lead to infection of the alveolar bone.

Thus, the indicated treatment of a carious lesion is to remove the infected tissue and necrotic dentin.

It is well known that current caries excavation procedures do not remove all bacteria from the cavity (Kidd et al. 2003, in Fejerskov& Kidd, "Dental Caries", Blackwell Munksgaard, Chapter 17). Therefore, the use of cavity disinfectants is often recommended especially in deep caries lesion to prevent pulpal infections. (Brännström et al. 1980, Caries Res 14: 276-284). Prevention of pulpal inflammation is also the reason why prior to pulp capping disinfection is indicated or why pulp-capping products were developed with antimicrobial properties.

Furthermore, disinfection is an integral part of endodontic therapy which is reflected in the statement that "endodontics is primarily a microbiological and then an anatomical and technical problem!" (Endodontie, Heinemann (ed.), Urban & Fischer 2001, p. 82). Especially problematic is the endodontic smear layer that is created during endodontic treatment and that hinders antibacterial substances to reach the bacteria in the smear layer and in the tubuli of the canal walls. Today, endodontic therapy often is not successful in killing the bacteria in the smear layer and in the tubuli which may cause recolonization (Endodontie, Heinemann (ed.), Urban & Fischer 2001, p. 89). Furthermore, current endodontic irrigants may compromise endodontic sealers and restorations (Sevcigan et al., IADR 2005, poster #2982).

During treatment of a carious tooth the dental practitioner may experience different treatments to become necessary: removal of necrotic dentin in shallower and deeper cavities and disinfection of the cavity. This disinfection of the cavity is especially advantageous in caries profunda situations where a pulpal infection is to be prevented. If the pulp is already infected it is necessary to remove the pulp and to disinfect and irrigate the endodontic region to prevent extensive infections.

The formulations disclosed in WO 2004/017988 remove infected dentin. They consist of at least one biologically active protease and/or at least one biologically active glycosidase. A further embodiment of the WO 2004/017988 contains water, acid, pepsin and a rheological additive.

EP 0884950 B1 and WO 00/27204 describe enzymes in combination with cationic polymers and N-hydroxyanilides respectively to kill bacteria.

US 2004/0071636 and WO 2004/000222 describe the use of bacteriophage derived lysozyme-like enzymes as anti-bacterial compounds.

It would, therefore, be desirable to have a product that specifically removes infected dentin and that contains agents to prevent bacterial contamination of the product.

This is to be solved by a dental composition comprising an enzyme according to claim 1. The enzyme of this composition has an enzyme activity at acidic pH values. It further contains a buffer providing an acidic pH value and one or more preservative agents, wherein at least one preservative agent is active at an acidic pH value.

The enzyme activity of the enzyme used in the present invention must not show the optimum activity at the acidic pH value that is provided by the buffer. Nevertheless it should have a total enzyme activity at that pH value that the buffer provides of at least 1 U/ml or more.

The enzyme comprising composition according to the invention can have, for example, total enzyme activities of from about 1 U to about 1,000,000 U per ml composition. The lower limit is, for example, about 5 or 10 U/ml wherein the inventive effect is normally considerably improved at a lower limit of the total enzyme activity of about 20 or about 30 or about 50 U/ml composition.

Preferred for this invention is a total enzyme activity of 500 to 50,000 U/ml of the composition or about 1,000 to about 8,000 U/ml of the composition. The desired activities can be obtained for example by adding in the case of pepsin used as enzyme an amount of about 0.5 to about 15 mg of pepsin of an ordinary, commercial available quality per ml composition.

The enzyme unit (abbreviated as U) is the respective amount of enzyme which is necessary to convert one µmol of a corresponding enzyme substrate per minute at standard conditions for the respective enzyme. The enzyme units as well as the standard conditions are known in the art. For example, in WO 2004/017988 which herein is in its completeness incorporated by reference, there are disclosed standard conditions for some type of enzymes.

In the special case of pepsin, for example, 1 Unit corresponds the amount of pepsin which releases 0.001 A280 as Trichloracetic acid soluble hydrolysis products from hemoglobin per minute at 37°C.

In the present case, the total enzyme activity [U/ml] relates to liquid as well as pasty and dry products which, for example, consist exclusively of a mixture of enzymes and optionally one or more adjuvants which are also dry.

The enzymes used in the present invention generally can be isolated from plants, animals or fungi as well as from bacteria or yeasts. They may have been produced also via bioengineering. Enzymes are suitable which were isolated from animals, bacteria or yeasts such as enzymes obtained from pig or chicken. Furthermore, also enzymes isolated from the bacterial genus *Streptomyces* or the fungus *Penicillium* or from the bacterial genus *Clostridium,* the fungus *Aspergillus* or *Triotirachium* are suitable. Enzymes are preferred which were isolated from *Streptomyces griseus, Clostridium histolyticum, Alicyclobacillus, Aspergillus nidulans, penicillium species* or *Tritirachium album.*

In the context of the present invention, the term "proteases" denotes all enzymes capable of hydrolyzing peptide bonds. Furthermore, this term denotes enzymes capable of hydrolyzing dipeptides, oligopeptides, polypeptides and proteins or derivatives thereof such as glycosylated derivatives.

The proteases used according to the invention are used for catalyzing the decomposition of protein components which may be present in a caries lesion. For this purpose, generally all proteases are suitable which catalyze protein and peptide degradation. However, in the context of the present invention, those proteases are especially suitable which are capable of catalyzing the decomposition of collagen fibers which are present, for example, in the material of the tooth, or which at least catalyze a change in the structure of the collagen fibers so that the collagen fibers have better solubility characteristics after the protease reaction.

A classification of proteases which are suitable according to the invention is also possible with regard to the amino acids or co-factors involved in the proteolytic catalysis. Thus, in the context of the present invention, generally all protease classes such as serine proteases, matrix metalloproteases, aspartate proteases, serine-carboxyl proteinases and cysteine proteases may be used. In general, the proteases comprised in the composition shall be used under conditions which allow the respective proteases to catalyze the degradation of the protein components which are present in a caries lesion.

In general, a composition according to the invention may comprise only one protease, but may comprise as well two or more different proteases.

According to one embodiment of the present invention a composition comprises, for example, one protease. According to another embodiment a composition according to the invention comprises about 1 to 10 different proteases, preferably 1 to 6, more preferably 1 to 4 and more preferably 2 to 3 and more preferably 2 different proteases.

According to another embodiment an inventive composition comprises only one protease, preferably an aspartate protease wherein pepsin and in particular pepsin from pig is preferred.

An enzyme comprising composition generally can have a pH value in the acidic range, i.e. a pH value of less than 7.0.

Preferably the pH value of an inventive dental composition is in the range of about pH 1 to about pH 4.

In this respect it is preferred that the enzyme of this invention has an enzyme activity at a pH value or a pH range of less than 4.0. It is not necessary that the enzyme has its maximum enzyme activity at this pH value but it should have at least 10 % of its maximal activity at a pH value of less than 4.0. Consequently the buffer should provide for a pH value in this range where the enzyme has an enzyme activity, i.e. for a pH value of less than 4.0.

In the context of the present invention, all customary buffer are suitable, such as phosphate buffer, carbonate buffer, acetate buffer, formate buffer, citrate buffer, tris buffer, glycylglycine buffer, glycine buffer or mixtures thereof. Sodium phosphate buffer, sodium hydrogen phosphate buffer, sodium dihydrogen phosphate buffer, potassium phosphate buffer, potassium hydrogen phosphate buffer, potassium dihydrogen phosphate buffer or pyrophosphate buffer are preferred. Suitable are also sodium carbonate buffer, potassium carbonate buffer, sodium hydrogen carbonate buffer or potassium hydrogen carbonate buffer. Especially preferably the inventive compositions comprise phosphate buffer and their components, respectively. An especially preferred phosphate buffer is a sodium dihydrogen phosphate buffer.

The buffer concentration preferably lies in the range of about 0.01 to about 2.0 M.

A composition according to the present invention can additionally contain preservatives. Generally, all types of preservatives can be used which inhibit the growth of microorganisms in such a composition and which are tolerated by the human body. It has, however, been found that conventional preservatives of the para hydroxy benzoic acid ester type are most preferred. Especially preferred are preservatives known under the names para hydroxy benzoic acid methyl ester (methylparabene) and para hydroxy benzoic acid propyl ester (propylparabene). Each of the named preservatives can be used as a sole preservative. It is, however, also possible to use combinations of such preservatives. The preservatives are generally used in an amount of about 0.001 to about 1 wt.-%, based on the weight of the composition. In a preferred embodiment of the present invention, preservatives are used in an amount of about 0.01 to about 0.25 wt.-%, based on the weight of the composition.

Additionally to the optional preservatives, the composition according to the present invention requires at least one preservative agent that is active at an acidic pH value and preferably is active at an pH value of less than 4.0. For example the preservative that is active at an acidic pH value could be an organic acid.

This preservative agent may be selected from the group of dichlorbenzylalcohol, phenylethylalcohol, sulfites, sorbic acid, dehydracetic acid dibromdicyanobutane, propionic acid, salicylic acid, formic acid, acetic acid, lactic acid, glutaraldehyde.

Preferably, the preservative agent of the present invention is benzoic acid or a derivative of benzoic acid or is a substance releasing benzoic acid including the salts of benzoic acid. More preferably, the preservative agent is benzoic acid.

By adding a preservative agent active at acidic pH values to the dental composition a certain preservation is obtained. Thus, the composition shows anti-bacterial properties and is protected against bacterial contamination. Additionally to this preservative effect a surprisingly synergistic effect is reached by the inventive composition. As known to a person skilled in the art the dental composition would according to the ingredients be useful to remove infected and/or necrotic dentin and/or an infected pulp. It would further be resistant against bacterial contamination. After testing the dental composition for its ability to reduce bacteria it was found that according to a synergistic effect the inventive composition reduces cariogenic bacteria by more than 5 log₁₀ levels in a very short time of treatment.

Thus, this composition is not only highly functional as a dental composition for caries removal or removal of pulp, pulpal residues, necrotic tissue and/or the endodontic smear layer in the root canal after endodontic treatment. It further exhibits protection against bacterial contamination. Additionally and due to its surprisingly effective antibacterial properties it can be used by the dental practitioner to disinfect the treated dental cavity after caries removal. According to its biologically compatibility the inventive composition shows an advantage over the compositions for caries removal or disinfection of the cavity known from the art. It is further possible to use the dental composition according to the present invention as an irrigation fluid for the endodontic region or for endodontic treatment and/or to disinfect the root canal and/or the tubuli of the canal walls after removal of the pulp. Therewith the endodontic smear layer is removed and the endodontic region is disinfected with one composition or in one step.

Particular advantageous is that the invention composition can be used for both steps explained above. Thus, the removal of necrotic tissue, carious tissue and infected dentin as well as removal of the pulp, pulpal residues and/or the endodontic smear layer in the root canal after endodontic treatment and the disinfection of the treated region is provided by only one composition. These treatments may be performed in only one step what is time saving over the treatment with compositions known from the art.

The composition according to the present invention may contain a rheological additive. As rheological additives, organic thickening agents are successfully used. These include but are not limited to starch, polyethylene glycol, polyvinylalcohol, polyvinyl pyrrolidone, xanthan, hydroxyethyl propylcellulose, hydroxybutyl methylcellulose, hydroxypropyl methycellulose, hydroxyethylcellulose and sodium carboxymethylcellulose. Even possible are inorganic thickening agents such as silica gels or phyllosilicates. Mixtures of two or more of the mentioned thickening agents may be used.

It is preferred, when a protease containing composition according to the present invention contains a polysaccharide as a rheological additive. Suitable polysaccharides are, for example, starch, mannan, xanthan, alginate, carragen, pectin, polyvinyl pyrrolidone, polyvinylalcohol, hydroxyethyl propylcellulose, hydroxybutyl methylcellulose, hydroxypropyl methycellulose, hydroxyethylcellulose or sodium carboxymethylcellulose and mixtures of two or more thereof.

A solvent may be used in the present dental composition. In general, each solvent which is able to dissolve the enzymes and other components without denaturing the enzyme and/or without interfering with other components of the present invention can be used. For example, all aqueous and organic solvents can be used that do not impair the activity of the enzymes to an extent that their use according to the invention is impossible. Suitable solvents are for example water, linear, branched or cyclic, saturated or unsaturated alcohols with 2 to about 10 C atoms, ketones, esters, carboxylic acids and mixtures of two or more of said types of solvents. Preferred solvents are water and water-alcohol mixtures.

According to the invention, for example dialkyl ketones or alcohols or polymerizable substances of low viscosity such as polyethylene glycol (PEG), hydroxyethyl methacrylate or (2,3-epoxypropyl) methacrylate and mixtures thereof can be used as solvents. Other suitable organic solvents are glycerin, dimethyl sulfoxide, tetrahydro furane, acetone, methyethyl ketone, cyclohexanol, toluene, methylen chloride, chloroform, alkanes and acetic acid alkyl esters, in particular acetic acid ethyl ester.

An inventive composition can comprise further adjuvants as disclosed in WO 2004/017988 such as complexing agents, enzyme substrates or enzyme effectors. According to the present invention, enzyme effectors comprise enzyme activators as well as enzyme inhibitors.

To enhance the storing properties of the composition it might be advantageous to provide the inventive composition with two components A and B, wherein component A comprises a solvent, an enzyme having an enzyme activity at acidic pH values and a buffer providing for a pH value above the pH value at which the enzyme is most active.

This component A can have a pH value in the poor acidic range. For example the pH value of this component A can be adjusted within the range of 4.5 to 6.5. Preferably, it can be adjusted in a range of about 5.0 to 6.0.

In this embodiment, component B comprises a solvent, a buffer system providing for a pH value equal to or less than the pH value at which the enzyme is most active and a preservative agent active at said pH value.

This component B can have a pH value in the strong acidic range. For example the pH value of this component B can be adjusted within the range of 1.0 to 4.0. Preferably, it can be adjusted in a range of about 1.5 to 3.5.

Thus, by the ability to choose different pH values for the components A and B some advantages are reached. The enzyme stability can be increased. It is further possible to add different preservative agents in the different components. Further, some additives show better solubility and/or efficiency at certain pH values. Thus, they can be added to that component A or B that has the more suitable pH value.

According to one embodiment of the present invention it is preferred that component A comprises water, an enzyme having an enzyme activity at acidic pH values, sodium phosphates and sodium hydroxide providing for a pH value above the pH value at which the enzyme is most active and a preservative agent and component B comprises water, sodium phosphates and phosphoric acid providing for a pH value equal to or less than the pH value at which the enzyme is most active and a preservative agent active at said pH value.

In a preferred embodiment according to the present invention, component A can contain the following constituents in the following amounts:

| | |
|---|---|
| pepsin: | about 0.1 to about 1.0 wt.-% |
| sodium phosphates: | about 0.6 to about 2.4 wt.-% |
| sodium hydroxide: | about 0.001 to about 0.5 wt.-% |
| hydroxyethyl cellulose: | about 0.2 to about 1.0 wt.-% |
| methyl parabene: | about 0.005 to about 0.05 wt.-% |
| propyl parabene: | about 0.005 to about 1.0 wt.-% |
| water: | ad 100 wt.-% |

In a preferred embodiment according to the present invention, component B can contain the following constituents in the following amounts:

| | |
|---|---|
| sodium phosphates: | about 10 to about 20 wt.-% |
| phosphoric acid: | about 5 to about 10 wt.-% |
| polyethylene glycol: | about 2 to about 5 wt.-% |
| hydroxyethyl cellulose: | about 0.2 to about 1.0 wt.-% |
| sodium benzoate or benzoic acid: | about 0.01 to about 2.0 wt.-% |
| water: | ad 100 wt.-% |

The dental composition according to the present invention may be used to remove carious dentin and/or necrotic tissue. In another embodiment of the invention it may be used to remove the pulp, the endodontic smear layer and/or pulpal residues after endodontic treatment such as removal of the pulp and preparation of the root canal. The inventive composition has an advantageous biological compatibility.

It may be further used to disinfect the dental cavity. This disinfection protects against reinfection of the cavity after the filling material is placed into the cavity and against a pulpal infection if the cavity is in an advanced state.

It may be further used as an endodontic irrigation fluid and/or to disinfect the root canal, the tubuli of the canal walls and/or the endodontic region.

### Examples:

Microbial disinfection procedures are considered effective when during the given treatment time more than 99.999% of the originally existing microorganisms are inactivated which corresponds to 5 log₁₀ levels (DGHM, List of Disinfectants, 2003). Thus, a caries removal system that within the given treatment time or faster reduces cariogenic bacteria by more than 5 log₁₀ levels is highly functional.

The antimicrobial action of the present invention was evaluated with acidogenic strains of the cariogenic genera Streptococcus and Lactobacillus. An experimental system was developed which allows to detect the reduction in bacterial levels of more than 5 log₁₀ levels. Streptococcus mutans DSM20523 and Lactobacillus paracaseii DSM 5622 were obtained from DSMZ, Braunschweig and stock cultures were generated by plating on Columbia/sheep blood-Agar (Oxoid) and Rogosa-Agar (Oxoid) respectively. Single colonies were used to inoculate 10 ml of Brain-Heart-Infusion (BHI) which was incubated at 37°C. 1-2 % of the 10 ml culture were used to inoculate 100 ml which was grown until stationary phase and concentrated by centrifugation.

Experiments were started by addition of 100 µl of concentrated bacterial suspension (S. mutans or L. paracasei) to 900 µl of the respective solution according to the examples. With the addition of the respective solution to the bacterial suspension the inactivation reaction started. The mixtures were equilibrated to 37°C in a thermostat. After the time intervals indicated samples of 100 µl were removed and mixed with nutrient broth to terminate the inactivation reaction. Control experiments showed that nutrient broth effectively terminated the inactivation reaction because no further decrease in Colony Forming Units ( = CFU) occurred.

Bacterial concentrations were determined as CFU by plating appropriate dilutions on agar plates.

The efficiency of the respective solution was measured by the reduction of CFU for each experiment. The reduction of bacteria is denoted in log₁₀ reductions. Log reductions were calculated by log₁₀ (CFU+1)t₀ - log₁₀ (CFU+1)tₓ (Paddick et al. 2003, Appl. Environ Microbiol 71: 2467-2472).

For each experiment to represents the sample at the starting time (t=0) and tₓ the sample at a given time point. This is depicted on the Y-axis of all graphs as "reduction (log₁₀ CFU /ml)".

### Example 1:

An aqueous solution A was prepared by mixing:

| ingredient | concentration [weight-%] |
|---|---|
| Sodium phosphates | 1.5 |
| Sodium hydroxide | < 0.1 % |
| Hydroxyethyl cellulose | 0.5 |
| Pepsin | 0.9 |
| methyl parabene | 0.07 |
| propyl parabene | 0.02 |
| Water | Ad 100 |

All ingredients except of sodium hydroxide were mixed together and the sodium hydroxide was than added drop by drop to adjust the solution A to a pH value of 5.5.

An aqueous solution B was prepared by mixing:

| ingredient | concentration [weight-%] |
|---|---|
| Sodium phosphates | 15 |
| Phosphoric acid | < 6 |
| Polyethylene glycol 200 | 4 |
| Hydroxyethyl cellulose | 0.5 |
| Sodium benzoate | 0.12 |
| Water | Ad 100 |

All ingredients except of phosphoric acid were mixed together and the phosphoric acid was than added slowly to adjust the solution B to a pH value of 2.1.

Both solutions A and B were mixed at a ratio of A:B of 1:3. The resulting dental composition (Ex.1) was used as follows in the examples 2, 3 and 4.

### Comparative Example 1

A comparative example (Comp.1) was prepared as follows:
An aqueous solution A' was prepared by mixing

| ingredient | concentration [weight-%] |
|---|---|
| Sodium phosphate | 1.5 |
| Sodium hydroxide | < 0.1 % |
| Water | Ad 100 |

All ingredients except of sodium hydroxide were mixed together and the sodium hydroxide was then added drop by drop to adjust the solution A' to a pH value of 5.5.

The solution B' was prepared by mixing:

| ingredient | concentration [weight-%] |
|---|---|
| Sodium phosphate | 15 |
| Phosphoric acid | < 6 |
| Water | Ad 100 |

All ingredients except of phosphoric acid were mixed together and the phosphoric acid was then added slowly to adjust the solution B' to a pH value of 2.1.

Both solutions A' and B' were mixed at a ratio of A':B' of 1:3. The resulting comparative composition (Comp.1) was used as follows in the example 2, 3.

### Example 2:

To show the antimicrobial action of Ex.1 and Comp.1 against cariogenic bacteria the following Example 2 was performed. Both solutions were added to concentrated bacterial suspension as explained above.

Values are shown as means (SD) where bacteria were detectable after the treatment. Values marked with ">" represent experiments where no bacteria could be cultivated after the treatment. Thus, the values marked "> 5" represent experiments where the killing reproducibly exceeded 5-log₁₀ units. "N/D" means not determined.

### Log₁₀-reduction of Ex.1 and Comp.1 against cariogenic bacteria [log₁₀ CFU/ml]

| L. paracasei | | | S.mutans | |
|---|---|---|---|---|
| t [min] | Ex.1 | Comp.1 | Ex.1 | Gomp.1 |
| 0.5 | 1.6 (0.32) | 0.25 (0.35) | N/D | N/D |
| 1 | > 5 | -0.07(0.51) | > 4.8 | -0.10(0.13) |
| 2 | > 5 | 0.27 (0.33) | > 5 | 0.27 (0.57) |
| 3 | N/D | N/D | > 5 | 0.65 (0.49) |
| 5 | > 5 | 1.46 (1.00) | > 5 | 1.25 (0.69) |

As can be seen from the data obtained here Ex.1 shows a strong bacterial reduction already after 1 minute. To the contrary the Comp.1 exhibits no bacterial reduction after 1 min and only a small reduction within the given time period of 5 minutes.

### Example 3:

The antimicrobial action of the single ingredients of Ex. 1 was determined separately for the following substances after 2 min (Ex.3a) and after 5 min (Ex.3b).:
Pep = Pepsin
PEG = Polyethylene glycol 200
Benz = Sodium benzoate
PHB = methyl/propyl parabenes

Thus, mixtures were prepared containing the ingredients of Comp.1 together with one of the indicated substances (Pep, PEG, Benz, PHB). The concentrations of each of the indicated substances were equal to those in Ex.1. The concentration of water according to Comp.1 was decreased accordingly.

In all experiments with a reduction greater than 5 log₁₀ units the limit of detection was reached. The log reduction is therefore greater than the value depicted.

### Ex.3a:

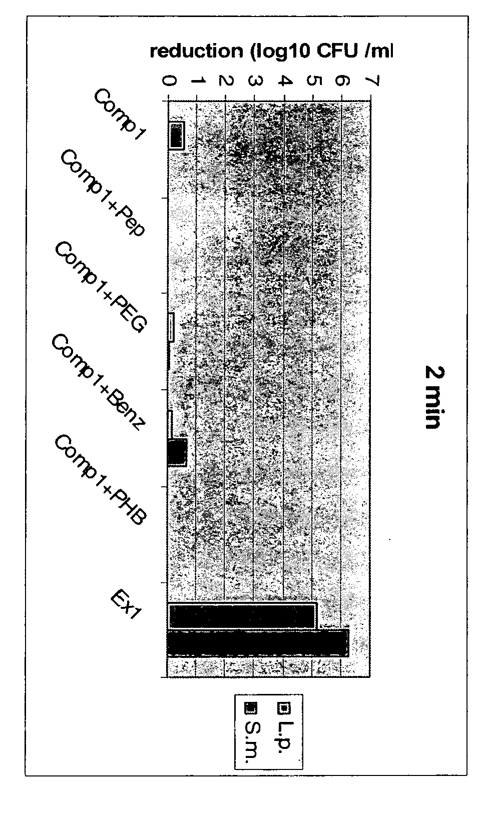

### Ex.3b:

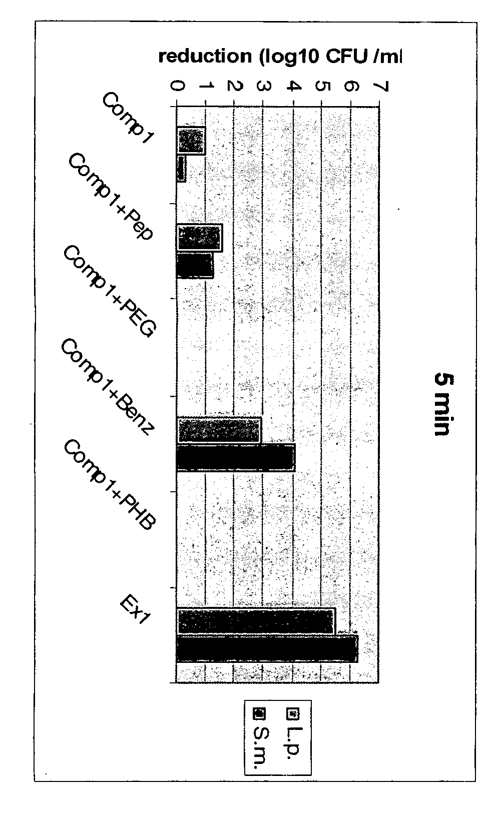

It can be seen from the results that after 2 minutes as well as after 5 minutes the antimicrobial action cannot be attributed to any single ingredient, rather the synergistic action of the formulation is the basis of the strong antibacterial effect.

### Comparative Example 2:

A comparative example (Comp.2) was prepared as follows:
An aqueous solution A" was prepared by mixing:

| ingredient | concentration [weight-%] |
|---|---|
| Sodium phosphate | 1.5 |
| Sodium hydroxide | < 0.1 % |
| Hydroxyethyl cellulose | 0.5 |
| Pepsin | 0.9 |
| methyl parabene | 0.07 |
| propyl parabene | 0.02 |
| Water | Ad 100 |

All ingredients except of sodium hydroxide were mixed together and the sodium hydroxide was then added drop by drop to adjust the solution A" to a pH value of 5.5.

The solution B" was prepared by mixing:

| ingredient | concentration [weight-%] |
|---|---|
| Sodium phosphate | 15 |
| Phosphoric acid | < 6 |
| Polyethylene glycol 200 | 4 |
| Hydroxyethyl cellulose | 0.5 |
| Water | Ad 100 |

All ingredients except of phosphoric acid were mixed together and the phosphoric acid was then added slowly to adjust the solution B" to a pH value of 2.0.

Both solutions A" and B" were mixed at a ratio of A":B" of 1:3. The resulting comparative composition (Comp.2) was used as follows in the example 4.

### Example 4:

Ex.1 was tested against Comp.2 as disclosed in WO 2004/017988. In all experiments marked with * the limit of detection was reached. The log₁₀ reduction is therefore greater than the value depicted.

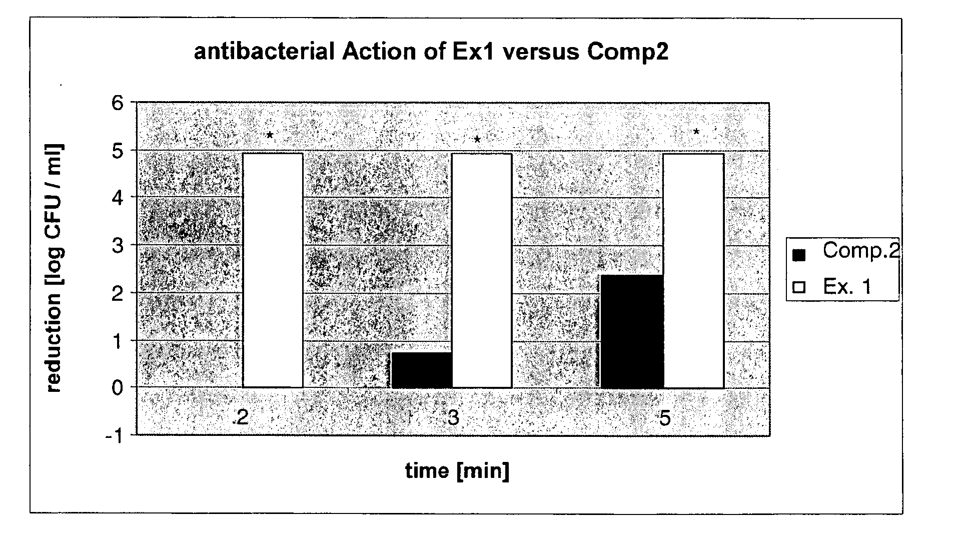

## Claims

1. A dental composition comprising an enzyme having an enzyme activity at acidic pH values, a buffer providing an acidic pH value and one or more preservative agents, wherein at least one preservative agent is active at acidic pH.

2. A dental composition according to claim 1 wherein the enzyme has an enzyme activity at a pH value or a pH range of less than 4.0 and a buffer providing for said pH value or said pH range.

3. A dental composition according to any of the preceding claims, wherein at least one preservative agent is benzoic acid or a derivative of benzoic acid or is releasing benzoic acid .

4. A dental composition according to any of the preceding claims wherein at least one preservative agent is benzoic acid.

5. A dental composition according to any of the preceding claims comprising a phosphate buffer.

6. A dental composition according to any of the preceding claims wherein the enzyme is selected from the group of proteases.

7. A dental composition according to any of the preceding claims wherein the enzyme is pepsin.

8. A dental composition according to any of the preceding claims additionally containing a rheological additive and/or a solvent and/or a preservative agent that is active at pH above 4.0.

9. A dental composition according to any of the preceding claims comprising sodium phosphates, phosphoric acid, sodium hydroxide, polyethylene glycol 200, hydroxethyl cellulose, pepsin, sodium benzoate, p-hydroxybenzoic acid methyl ester, p-hydroxybenzoic acid propyl ester and water.

10. A. dental composition comprising two components A and B, wherein
component A comprises a solvent, an enzyme having an enzyme activity at acidic pH values and a buffer providing for a pH value above the pH value at which the enzyme is most active and
component B comprises a solvent, a buffer providing for a pH value equal to or less than the pH value at which the enzyme is most active and a preservative agent active at said pH value.

11. A dental composition according to claim 10 wherein component A comprises a buffer providing for a pH value of about 5 to 6 and wherein component B comprises a buffer providing for a pH value of about 1.5 to 3.5.

12. A dental composition according to claim 10 or 11 comprising two components A and B, wherein
component A comprises water, an enzyme having an enzyme activity at acidic pH values, sodium phosphates and sodium hydroxide providing for a pH value above the pH value at which the enzyme is most active and a preservative agent
component B comprises water, sodium phosphates and phosphoric acid providing for a pH value equal to or less than the pH value at which the enzyme is most active and a preservative agent active at said pH value.

13. A dental composition according to any of the claims 10 to 12, wherein at least one preservative agent is benzoic acid or a derivative of benzoic acid or is releasing benzoic acid .

14. A dental composition according to any of the preceding claims wherein the solution is used to remove carious dentin and/or to disinfect the dental cavity.

15. A dental composition according to any of the preceding claims wherein the solution is used as an endodontic irrigation fluid and/or to disinfect the root canal.
